# EUROPEAN PATENT APPLICATION

(11) **EP 2 810 647 A1**
(43) Date of publication of application: **10.12.2014**
(21) Application number: 13002931.7
(22) Date of filing: 06.06.2013
(51) Int. Cl.: A61K 31/165, A61K 47/12, A61P 25/20, A61P 25/22, A61P 25/24

(54) **Pharmaceutical formulations comprising agomelatine in the form of agomelatine co-crystal with an organic acid**

(71) Applicant: Zentiva, a.s., 102 37 Praha 10 (CZ)
(72) Inventor: Rezac, Jaroslav, 277 11 Neratovice (CZ); Novak, Martin, 101 00 Praha 10 (CZ); Pribyl, Daniel, 150 00 Praha 5 (CZ); Hanovska, Anna, 149 00 Praha 11 (CZ); Ridvan, Ludek, 102 00 Praha 10 (CZ); Dumicic, Aleksandra, 10 000 Zagreb (HR); Kral, Vladimir, 120 00 Praha 2 (CZ)
(74) Representative: Jirotkova, Ivana

(57) **Abstract**

A pharmaceutical formulation, comprising agomelatine in the form of agamelatine co-crystal with a co-crystal former selected from the group of organic acids in an amount corresponding to 15 - 22 mg agomelatitte dose per one dosage unit and at least one pharmaceutically acceptable excipient for effective treatment of diseases responsive to therapy by agomelatine.

## Description

### Field of the invention

The present invention relates to immediate-release pharmaceutical formulations comprising agomelatine in the form of agomelatine co-crystal with an organic acid which provide effective treatment of diseases responsive to therapy by agomelatine at significantly lower agomelatine dose in comparison with present common practice. Agomelatine is known to be effective e.g. for the treatment of major depression, seasonal affective disorder, sleep disorders, anxiety disorders, mood disorders, cardiovascular pathologies, pathologies of the digestive system, insomnia and fatigue due to jet-lag, appetite disorders and obesity.

### Background of the invention

Agomelatine (N-[2-(7-methoxy-1-naphthalenyl)ethyl]acetamide), its preparation and its pharmaceutical use in treatment of stress, sleep disorders, anxiety seasonal depression, insomnia and tiredness due to "jet lag", schizophrenia, panic attacks, melancholy, the regulation of appetite, insomnia etc. were first described in the priority patent application FR2658818. The effective dose was suggested to vary according to the age and weight of the patient, the route of administration or the indications for treatments within a range of 0.1 mg to 1 g in 24 hours. A tablet consisting of 50 mg agomelatine, derivative (N-[2-(7-methoxy-1-naphthalenyl)ethyl]butyramide), wheat starch, corn starch, lactose, magnesium stearate, silica and hydracypropylcellulose was disclosed.

At present, agomelatine is registered and marketed by Les Laboratoires Servier Company under a trade name Valdoxan® and it is indicated for treatment of major depressive episodes in adults. The recommended dose of agomelatine is 25 mg once daily taken orally and if there is no improvement of symptoms, the dose may be increased to 50 mg once daily, i.e. two 25 mg tablets. The Valdoxan® tablets are film-coated tablets with tablet cores consisting of agomelatine, lactose monohydrate, maize starch, povidon, sodium carboxymethyl starch, stearic acid, magnesium stearate and anhydrous colloidal silica which are coated by a coat consisting of hypromelose, glycerol, makrogol, magnesium stearate and colourants (yellow ferric oxide, titanium oxide and black ink). Agomelatine is present in the polymorphic form II in Valdoxan®.

Use of agomelatine in obtaining medicaments intended for the treatment of Generalized Anxiety Disorder was disclosed in patent family of WO2007116136 (Les Laboratoires Servier). The useful dosages were described as varying between 1 mg and 50 mg of agomelatine per day. A pharmaceutical composition was disclosed, consisting of 25 mg agomelatine, lactose monohydrate, magnesium stearate, povidone, anhydrous colloidal silica, cellulose sodium glycolate and stearic acid. A clinical study with a 25 mg agomelatine per day was carried out, and the dose was increased to 50 mg agomelatine per day in cases of low response after treatment for two weeks.

Similarly to above mentioned WO2007116136 (Les Laboratoires Servier), the same pharmaceutical formulation and the same dosing (25 mg, respectively 50 mg per day) were used in a clinical study on the treatment of sleep disorders among depressed patients (WO2007028905 by Les Laboratoires Servier). Furthermore, the same pharmaceutical formulation and dose of 25 mg with possibility of increasing to 50 mg per day was suggested for the treatment of periventricular leukomalacia (WO2008071869 by Les Laboratoires Servier) and for the treatment of Smith-Magenis syndrome (WO2008071870 by Les Laboratoires Servier).

Different types of oral dosage forms were described in the prior art documents. A general controlled release pharmaceutical composition, where agomelatine is mentioned as one of the possible active ingredients, is described in EP1064935 (Les Laboratoires Servier). A solid orodispersible pharmaceutical composition of agomelatine is described in WO03061644 and WO2007068829 (Les Laboratoires Servier). A capsule composition of agomelatinu, mannitol, silica and pre-gelatinized starch was disclosed in CN101991559 (Tianjin Hankang Pharmaceutical Biotechnology).

A method for preparation of a solid pharmaceutical formulations by wet granulation is described in WO2011079608 (Shanghai Zhongxi Pharmaceutical Corporation). Agomelatine (25 mg) was dissolved in a acidifier-containing acid solution (ethanol and hydrochloric acid), homogeneously mixed with an alkalizer (natrium hydroxide) and pharmaceutically excipients (povidone). This granulation liquid was used in a fluid spray granulator for granulation of lactose and starch. Carboxymethyl starch sodium and magnesium stearate were admixed after wet granulation and the mixture was compressed into tablet cores which were then film-coated.

There were described many polymorhic forms of agomelatine, e.g. form II (patent family of WO05077887 by Les Laboratoires Servier), form III (patent family of WO2007015003 by Les Laboratoires Servier), form IV (patent family of WO2007015002 by Les Laboratoires Servier), form V (patent family of WO2007015004 by Les Laboratoires Servier), form VI (patent family of WO2009095555 by Les Laboratoires Servier).

As stated above, the polymorphic form II was disclosed in the patent application WO05077887 (Les Laboratoires Servier). In this patent application were described formulations comprising 25 mg agomelatine of polymorphic form II and further consisting of filler (lactose monohydrate, maltodextrin), lubricants (magnesium stearate and stearic acid), binders (pregelatinized starch or povidone), disintegrants (starch or cellulose sodium glycolate), glidants (anhydrous colloidal silica). The polymorphic form II was lately disclosed as a preferred one in all the patent applications on the new medical uses of agomelatine (WO2007116136, WO2007028905, WO2008071869, WO2008071870, all by Les Laboratoires Servier).

Besides the polymorphic forms of agomelatine, co-crystals of agomelatine were disclosed in the prior art documents. The term "cocrystal" or "co-crystal" is understood to be a binary molecular crystal containing the molecules of the active pharmaceutical ingredient together with another molecular species in a defined stoichiometric ratio where both components are in their neutral state. The terms "cocrystal" and "co-crystal" are generally understood to be synonymous terms referring to such a system. The second component in the co-crystal (the component other than the active pharmaceutical ingredient) is commonly referred to as a "co-crystal former". Generally, pharmaceutically acceptable co-crystal formers include any molecule considered acceptable as a counterion for a pharmaceutical salt or known as a pharmaceutical excipient. A widely accepted definition of a pharmaceutical co-crystal is a crystalline system containing an active pharmaceutical molecule and a co-crystal former that is a solid at ambient temperature and pressure in a defined stoichiometric ratio, although a cocrystal is not limited to containing only two components. The components of the cocrystal are linked by hydrogen bonding and other non-covalent and non-ionic interactions (Aakeroy and Salmon, CrystEngComm, 2005, 439-448). This definition distinguishes co-crystals from crystalline solvates, in which case one of the components is a liquid at ambient temperature and pressure.

Co-crystals of agomelatine were disclosed in patent applications WO2012146371 (Zentiva) and WO2012168665 (Les Laboratoires Servier).

The earlier patent application WO2012146371 (Zentiva) discloses the co-crystals of agomelatine with a co-crystal former possessing at least two carboxylic or at least one sulfonic group in its molecule and methods of preparing these co-crystals which can be used in the preparation of pharmaceutical formulations for the treatment of depression or major depressive disorder. These co-crystals could be prepared reproducibly and had bioavailability, stability, hygroscopicity and mechanical properties that make them suitable for use in the preparation of pharmaceutical formulations that can satisfy the regulations in force governing the quality of pharmaceutical preparations. Co-crystals with citric acid, maleic acid or benzenesulfonic acid and methods of their preparation are specified in the examples.

The later patent application WO2012168665 (Les Laboratoires Servier) discloses co-crystals of agomelatine with organic acids (e.g. with para-hydroxybenzoic acid, citric acid, oxalic acid, gallic acid, maleic acid, malonic acid, glutaric acid, glycolic acid and ketoglutaric acid) and their preparation. The co-crystals were reported to be advantageous for usage in the pharmaceutical formulations because it was possible to modify (promote or reduce) the dissolution rate ofagomelatine in comparison to the marketed product Valdoxan®. The co-crystals according to this patent application can be used for preparation of pharmaceutical formulations for oral, parenteral or nasal administration, such as different types of tablets, granules, capsules, lozenges, suppositories, creams, ointments, dermal gels, injections etc. The dosage can, according to the disclosure of the patent application, vary from 0.1 mg to 1 g of agomelatine per day in one or more administrations. An immediate-release tablet is disclosed, consisting of co-crystal of agomelatine with para-hyroxybenzoic acid (corresponding to 25 mg agomelatine), lactose monohydrate, magnesium stearate, maize starch, maltodextrin, anhydrous colloidal silica and pregelatinised maize starch. A retarded-release tablet, consisting of agolematine co-crystal with ketoglutaric acid (corresponding to 25 mg agomelatine), lactose monohydrate, magnesium stearate, povidone, anhydrous colloidal silica and hypromellose, is disclosed as well.

### Summary of the invention

The present invention relates to immediate released pharmaceutical formulations comprising agomelatine in the form of co-crystal with an organic acid which provide effective treatment of diseases responsive to therapy by agomelatine at significantly lower agomelatine doses in comparison with present common practice. This finding was particularly surprising given that there was shown no difference in the dissolution profiles and in the release rates of agomelatine from the tested formulation and from the commercially used formulation.

Formulations for treating diseases responsive to therapy by agomelatine, in particular for treating major depressive episodes, generalized anxiety disorder, sleep disorders among depressed patients, treatment of periventricular leukomalacia and Smith-Magenis syndrome are disclosed. Formulations with significantly increased *in-vivo* bioavailability in comparison to the marketed product Valdoxan® permit effective treatment of patients at lower doses of agomelatine than previously described, without compromising the therapeutic effect.

Agomelatine, when absorbed from intestine, undergoes extensive metabolization in liver by cytochrome P450 1A2 oxidase (CYP 1A2) on inactive metabolites. However, several important endogenous substances, such as arachichonic acid derivatives, sexual hormone derivatives or melatonin, are also substrates for CYP 1A2. Arachichonic acid derivatives constitute large group of signalling molecules involved in fine tuning of inflammatory response, platelets aggregation, and muscle growth. Impairment of arachidonic acid metabolism contributes to development of Alzheimer's disease and bipolar disorder. Hormonal contraceptives which are derivatives of sexual hormones are known inducers of CYP 1A2. Melatonin is the major messenger of circadian rhythms in the body and is metabolized by CYP 1A2.

It is therefore desirable to reduce the interference of medication with CYP 1A2. Enhanced absorption of agomelatine will allow reducing the agomelatine dose without compromising the therapeutic effect.

The object of the invention is pharmaceutical formulation comprising agomelatine in the form of an agomelatine co-crystal with a co-crystal former form the group of organic acids and at least one pharmaceutically acceptable excipient for effective treatment of diseases responsive to therapy by agomelatine, wherein the amount of the agamelatine co-crystal in the formulation corresponds to 15 - 22 mg of agomelatine per one dosage unit. The preferred co-crystal formers are citric acid, maleic acid and benzenesulfonic acid. Especially preferred is a co-crystal of agomelatine with citric acid in molar ratio 1:1.

The pharmaceutical forms comprising the co-crystals according to the invention will be used for their activity in respect of the central nervous system and microcirculation, in the treatment of stress, sleep disorders, anxiety disorders and especially generalised anxiety disorder, obsessive-compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders. In another field of activity, it will be possible to use the co-crystals according to the invention in sexual dysfunctions, as ovulation inhibitors and immunomodulators and in the treatment of cancers.

The co-crystals according to the invention will preferably be used in treatments for major depression, seasonal affective disorder, sleep disorders, anxiety disorders, mood disorders, cardiovascular pathologies, pathologies of the digestive system, insomnia and fatigue due to jet-lag, appetite disorders and obesity.

### Brief description of the figures

Figure 1: Dissolution profiles of two batches of formulation according to present invention (line 1 and line 3) and two batches of marketed Valdoxan® formulations (line 2 and line 4) at pH 2.0.
Figure 2: Dissolution profiles of two batches of formulation according to present invention (line 1 and line 4) and two batches of marketed Valdoxan® formulations (line 2 and line 3) at pH 4.5,
Figure 3: Dissolution profiles of two batches of formulation according to present invention (line 1 and line 3) and two batches of marketed Valdoxan® formulations (line 2 and line 4) at pH 6.8

### Detailed description

The present application discloses formulations of agomelatine with surprisingly high *in-vivo* bioavailability comprising agomelatine in the form of co-crystal with organic acids.

It was found that formulations comprising agomelatine in the form of co-crystal with organic acids, especially with the citric acid, preferably in the molar ratio of agomelatine to co-crystal former 1:1, provide significantly higher bioavailability *in-vivo* in comparison to the marketed formulation of agomelatine Valdoxan®.

The present invention embraces, in certain aspects, use of agomelatine in the form of co-crystal with organic acids, especially with the citric acid, preferably in the molar ratio of agomelatine to co-crystal former 1:1, at agomelatine doses significantly lower than those previously shown to be effective in treating diseases responsive to therapy by agomelatine, wherein the significantly lower dosage is therapeutically effective.

In another embodiment, the present invention embraces, in certain aspects, use of agomelatine in the form of co-crystal with organic acids, especially with the citric acid, preferably in the molar ratio of agomelatine to co-crystal former 1:1, at doses significantly lower than those previously shown to be effective in treating major depressive episodes, wherein the significantly lower dosage is therapeutically effective.

In another embodiment, the present invention embraces, in certain aspects, use of agomelatine in the form of co-crystal with organic acids, especially with the citric acid, preferably in the molar ratio of agomelatine to co-crystal former 1:1, at doses significantly lower than those previously shown to be effective in treating generalized anxiety disorder, wherein the significantly lower dosage is therapeutically effective.

In another embodiment, the present invention embraces, in certain aspects, use of agomelatine in the form of co-crystal with organic acids, especially with the citric acid, preferably in the molar ratio of agomelatine to co-crystal former 1:1, at doses significantly lower than those previously shown to be effective in treating sleep disorders among depressed patients, wherein the significantly lower dosage is therapeutically effective.

In another embodiment, the present invention embraces, in certain aspects, use of agomelatine in the form of co-crystal with organic acids, especially with the citric acid, preferably in the molar ratio of agomelatine to co-crystal former 1:1, at doses significantly lower than those previously shown to be effective in treatment of periventricular leukomalacia, wherein the significantly lower dosage is therapeutically effective.

In another embodiment, the present invention embraces, in certain aspects, use of agomelatine in the form of co-crystal with organic acids, especially with the citric acid, preferably in the molar ratio of agomelatine to co-crystal former 1:1, at doses significantly lower than those previously shown to be effective in treating Smith-Magenis syndrome, wherein the significantly lower dosage is therapeutically effective.

In another embodiment, the invention embraces administration of lower doses of agomelatine in the form of co-crystal with organic acids, especially with the citric acid, preferably in the molar ratio of agomelatine to co-crystal former 1:1, in order to treat diseases responsive to therapy by agomelatine.

In another embodiment, the invention embraces administration of lower doses of agomelatine in the form of co-crystal with organic acids, especially with the citric acid, preferably in the molar ratio of agomelatine to co-crystal former 1:1, in order to treat major depressive episodes.

In another embodiment, the invention embraces administration of lower doses of agomelatine in the form of co-crystal with organic acids, especially with the citric acid, preferably in the molar ratio of agomelatine to co-crystal former 1:1, in order to treat generalized anxiety disorder.

In another embodiment, the invention embraces administration of lower doses of agomelatine in the form of co-crystal with organic acids, especially with the citric acid, preferably in the molar ratio of agomelatine to co-crystal former 1:1, in order to treat sleep disorders among depressed patients.

In another embodiment, the invention embraces administration of lower doses of agomelatine in the form of co-crystal with organic acids, especially with the citric acid, preferably in the molar ratio of agomelatine to co-crystal former 1:1, in order to treat periventricular leukomalacia.

In another embodiment, the invention embraces administration of lower doses of agomelatine in the form of co-crystal with organic acids, especially with the citric acid, preferably in the molar ratio of agomelatine to co-crystal former 1:1, in order to treat Smith-Magenis syndrome.

In one embodiment, the present invention embraces the use of agomelatine in the form of co-crystal with organic acids, especially with the citric acid, preferably in the molar ratio of agomelatine to co-crystal former 1:1, at agomelatine doses reduced by 35-40% compared to those previously shown to be effective in treating diseases responsive to therapy by agomelatine.

In one embodiment, the present invention embraces the use of agomelatine in the form of co-crystal with organic acids, especially with the citric acid, preferably in the molar ratio of agomelatine to co-crystal former 1;1, at agomelatine doses reduced by 35-40% compared to those previously shown to be effective in treating major depressive episodes.

In one embodiment, the present invention embraces the use of agomelatine in the form of co-crystal with organic acids, especially with the citric acid, preferably in the molar ratio of agomelatine to co-crystal former 1:1, at agomelatine doses reduced by 35-40% compared to those previously shown to be effective in treating generalized anxiety disorder.

In one embodiment, the present invention embraces the use of agomelatine in the form of co-crystal with organic acids, especially with the citric acid, preferably in the molar ratio of agomelatine to co-crystal former 1:1, at agomelatine doses reduced by 35-40% compared to those previously shown to be effective in treating sleep disorders among depressed patients.

In one embodiment, the present invention embraces the use of agomelatine in the form of co-crystal with organic acids, especially with the citric acid, preferably in the molar ratio of agomelatine to co-crystal former 1:1, at agomelatine doses reduced by 35-40% compared to those previously shown to be effective in treating periventricular leukomalacia.

In one embodiment, the present invention embraces the use of agomelatine in the form of co-crystal with organic acids, especially with the citric acid, preferably in the molar ratio of agomelatine to co-crystal former 1:1, at agomelatine doses reduced by 35-40% compared to those previously shown to be effective in treating Smith-Magenis syndrome.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat diseases responsive to therapy by agomelatine.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat diseases responsive to therapy by agomelatine.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat diseases responsive to therapy by agomelatine.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat major depressive episodes.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat major depressive episodes.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat major depressive episodes.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat generalized anxiety disorder.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat generalized anxiety disorder.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat generalized anxiety disorder.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat sleep disorders among depressed patients.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat sleep disorders among depressed patients.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat sleep disorders among depressed patients.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat periventricular leukomalacia.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat periventricular leukomalacia.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat periventricular leukomalacia.

In another embodiment, the invention embraces administration of agomelatine in the form of co-crystal with organic acids, especially with the citric acid, preferably in the molar ratio of agomelatine to co-crystal former 1:1 in an amount corresponding to 15-22 mg agomelatine, in order to treat Smith-Magenis syndrome.

Preferably, the invention embraces administration of agomelatine in the form of co-crystal with organic acids, especially with the citric acid, preferably in the molar ratio of agomelatine to co-crystal former 1:1 in an amount corresponding to 16-20 mg agomelatine, in order to treat Smith-Magenis syndrome.

Still more preferably, the invention embraces administration of agomelatine in the form of co-crystal with organic acids, especially with the citric acid, preferably in the molar ratio of agomelatine to co-crystal former 1:1 in an amount corresponding to 18 mg agomelatine, in order to treat Smith-Magenis syndrome.

In another embodiment, the invention embraces use of a reduced dose of agomelatine for the manufacture of a pharmaceutical formulation for treating diseases responsive to therapy by agomelatine, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for treating major depressive episodes, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for treating generalized anxiety disorder, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for treating sleep disorders among depressed patients, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for periventricular leukomalacia, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for Smith-Magenis syndrome, wherein the agomelatine in said pharmaceutical formulation is in the form of co-crystal and wherein the reduced dose of agomelatine is within 15 to 22 mg, preferably within 16 to 20 mg and still more preferably the dose of agomelatine is 18 mg.

In another embodiment, the invention embraces use of a reduced dose of agomelatine for the manufacture of a pharmaceutical formulation for treating diseases responsive to therapy by agomelatine, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention embraces use of a reduced dose of agomelatine for the manufacture of a pharmaceutical formulation for treating major depressive episodes, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention embraces use of a reduced dose of agomelatine for the manufacture of a pharmaceutical formulation for treating generalized anxiety disorder, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention embraces use of a reduced dose of agomelatine for the manufacture of a pharmaceutical formulation for treating sleep disorders among depressed patients, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention embraces use of a reduced dose of agomelatine for the manufacture of a pharmaceutical formulation for periventricular leukomalacia, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention embraces use of a reduced dose of agomelatine for the manufacture of a pharmaceutical formulation for Smith-Magenis syndrome, wherein the agomelatine in said pharmaceutical formulation is in the form of co-crystal, wherein the reduced dose of agomelatine is within 15 to 22 mg, preferably within 16 to 20 mg and still more preferably the dose of agomelatine is 18 mg and wherein the dose of agomelatine is to be administered in one daily dose.

In another embodiment, the invention encompasses a method of increasing the bioavailability of agomelatine by converting said agomelatine to an agomelatine co-crystal with organic acids, especially with the citric acid, preferably in the molar ratio of agomelatine to co-crystal former 1:1 and administering said co-crystal composition to a subject in need thereof.

Yet in another embodiment, the invention encompasses a method of increasing the bioavailability of agomelatine by converting said agomelatine to an agomelatine co-crystal with organic acids, especially with the citric acid, preferably in the molar ratio of agomelatine to co-crystal former 1:1 and administering said co-crystal composition to a subject in need thereof, wherein said composition provides an increase in bioavailability of the agomelatine co-crystal as measured by AUC₀₋ₜ of at least 35% relative to dosing of agomelatine.

Yet in another embodiment, the invention encompasses a method of increasing the bioavailability of agomelatine by converting said agomelatine to an agomelatine co-crystal with organic acids, especially with the citric acid, preferably in the molar ratio of agomelatine to co-crystal former 1:1 and administering said co-crystal composition to a subject in need thereof, wherein said increase in bioavailability of the agomelatine is measured in blood plasma.

In another embodiment, the invention encompasses a method of increasing the effectiveness of agomelatine by converting said agomelatine to an agomelatine co-crystal with organic acids, especially with the citric acid, preferably in the molar ratio of agomelatine to co-crystal former 1:1 and administering said co-crystal composition to a subject in need thereof.

Yet in another embodiment, the invention encompasses a method of increasing the effectiveness of agomelatine by converting said agomelatine to an agomelatine co-crystal with organic acids, especially with the citric acid, preferably in the molar ratio of agomelatine to co-crystal former 1:1 and administering said co-crystal composition to a subject in need thereof, wherein said effectiveness of the agomelatine is measured in blood plasma.

Another embodiment of the invention encompasses a composition comprising agomelatine co-crystal with an organic acid, especially with the citric acid, preferably in the in the molar ratio of agomelatine to co-crystal former 1:1 and one or more pharmaceutically acceptable excipients.

Another embodiment of the invention encompasses a composition comprising agomelatine co-crystal with an organic acid, especially with the citric acid, preferably in the in the molar ratio of agomelatine to co-crystal former 1:1 and a filler, a disintegrant, a binder, a glidant and a lubricant.

Another embodiment of the invention encompasses a composition comprising agomelatine co-crystal with an organic acid, especially with the citric acid, preferably in the in the molar ratio of agomelatine to co-crystal former 1:1 and one or more pharmaceutically acceptable excipients, wherein the bioavailability of the agomelatine co-crystal measured by AUC₀₋ₜ is increased by at least 35% relative to dosing agomelatine.

Another embodiment of the invention encompasses a composition comprising agomelatine co-crystal with an organic acid, especially with the citric acid, preferably in the in the molar ratio of agomelatine to co-crystal former 1:1 and one or more pharmaceutically acceptable excipients, wherein the bioavailability of the agomelatine co-crystal measured by Cₘₐₓ is increased by at least 35% relative to dosing agomelatine.

In another embodiment, the invention encompasses a pharmaceutical formulation comprising agomelatine in the form of agomelatine co-crystal with a co-crystal former selected from the group of organic acids wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a preferred embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine,

In a more preferred embodiment, the invention encompasses a pharmaceutical formulation comprising agomelatine in the form of agomelatine co-crystal with a co-crystal former of citric acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In another preferred embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a more preferred embodiment, the invention encompasses a pharmaceutical formulation comprising agomelatine in the form of agomelatine co-crystal with a co-crystal former of citric acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a preferred embodiment, the invention encompasses a pharmaceutical formulation comprising agomelatine in the form of agomelatine co-crystal with a co-crystal former of citric acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine.

In a more preferred embodiment, the invention encompasses a pharmaceutical formulation comprising agomelatine in the form of agomelatine co-crystal with a co-crystal former of citric acid with the molar ratio of agomelatine to the co-crystal former being 1:1wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine. In a preferred embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In a more preferred embodiment, the invention encompasses a pharmaceutical formulation comprising agomelatine in the form of agomelatine co-crystal with a co-crystal former of citric acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective In treatment of major depressive episodes, generalized anxiety disorder, sleep disorders among depressed patients, periventricular leukomalacia and/or Smith-Magenis syndrome.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1.1 wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In another preferred embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In a more preferred embodiment, the invention encompasses a pharmaceutical formulation comprising agomelatine in the form of agomelatine co-crystal with a co-crystal former of citric acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1wherein the amount of said co-crystal corresponds to 16-20 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In a preferred embodiment, the invention encompasses a pharmaceutical formulation comprising agomelatine in the form of agomelatine co-crystal with a co-crystal former of citric acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In a more preferred embodiment, the invention encompasses a pharmaceutical formulation comprising agomelatine in the form of agomelatine co-crystal with a co-crystal former of citric acid with the molar ratio of agomelatine to the co-crystal former being 1:1wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of major depressive episodes, generalized anxiety disorder, sleep disorders among depressed patients, periventricular leukomalacia and/or Smith-Magenis syndrome.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1;1 wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1 where in the amount of said co-crystal corresponds to 16-20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1 wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1 wherein the amount of said co-crystal corresponds to 15 - 22 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 16 - 20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1:1 wherein the amount of said co-crystal corresponds to 16 -20 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

in another embodiment, the invention encompasses a pharmaceutical formulation comprising agomelatine in the form of agomelatine co-crystal with a co-crystal former selected from the group of citric acid, maleic acid and benzenesulfonic acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in in two daily doses.

In a more preferred embodiment, the invention encompasses a pharmaceutical formulation comprising agomelatine in the form of agomelatine co-crystal with a co-crystal former of citric acid wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in in two daily doses.

In a still more preferred embodiment, the invention encompasses a pharmaceutical formulation comprising agomelatine in the form of agomelatine co-crystal with a co-crystal former of citric acid with the molar ratio of agomelatine to the co-crystal former being 1;1 wherein the amount of said co-crystal corresponds to 18 mg agomelatine dose per one dosage unit and wherein said pharmaceutical formulation further comprises at least one pharmaceutically acceptable excipient and wherein the pharmaceutical formulation is effective in treatment of diseases responsive to therapy by agomelatine when administered in two daily doses.

The pharmaceutical formulations comprising the co-crystals according to the invention will be used for their activity in respect of the central nervous system and microcirculation, in the treatment of stress, sleep disorders, anxiety disorders and especially generalised anxiety disorder, obsessive-compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders. In another field of activity, it will be possible to use the co-crystals according to the invention in sexual dysfunctions, as ovulation inhibitors and immunomodulators and in the treatment of cancers.

The co-crystals according to the invention will preferably be used in treatments for major depression, seasonal affective disorder, sleep disorders, anxiety disorders, mood disorders, cardiovascular pathologies, pathologies of the digestive system, insomnia and fatigue due to jet-lag, appetite disorders and obesity.

### Definitions

The term "low dose" or "lower dose" or "reduced dose" of agomelatine are meant relatively to the doses of agomelatine previously described to be effective in treating diseases responsive to therapy by agomelatine.

The term "agomelatine co-crystal" is meant agomelatine co-crystal with organic acid.

The term "cocrystal" or "co-crystal" is understood to be a binary molecular crystal containing the molecules of the active pharmaceutical ingredient together with another molecular species in a defined stoichiometric ratio where both components are in their neutral state. The terms "cocrystal" and "co-crystal" are generally understood to be synonymous terms referring to such a system. The second component in the co-crystal (the component other than the active pharmaceutical ingredient) is commonly referred to as a "co-crystal former". A widely accepted definition of a pharmaceutical co-crystal is a crystalline system containing an active pharmaceutical molecule and a co-crystal former that is a solid at ambient temperature and pressure in a defined stoichiometric ratio, although a co-crystal is not limited to containing only two components. The components of the co-crystal are linked by hydrogen bonding and other non-covalent and non-ionic interactions (Aakeroy and Salmon, CrystEngComm, 2005, 439-448). This definition distinguishes co-crystals from crystalline solvates, in which case one of the components is a liquid at ambient temperature and pressure.

The preferred co-crystal formers of the present invention are citric acid, maleic acid or benzenesulfonic acid.

Still more preferred co-crystal former is citric acid. The preferred molar ratio of agomelatine to the co-crystal former citric acid is 1:1.

The term "effective treatment" means a treatment that brings the same level of therapeutic effect as the treatment previously shown to be effective, i.e. the same therapeutic effect that was described e.g. in the patent applications WO2007116136, WO2007028905, WO2008071869, WO2008071870 (Les Laboratoires Servier) or in the Summary of Product Characteristic of the marketed product Valdoxan®, or which can be achieved by the marketed product Valdoxan®.

The term "effective dose" is a dose that, when administered, results in the effective treatment to a subject in need thereof.

### Examples

### The in-vitro dissolutions of formulations compising agomelatine

The description part of the patent application WO2012168665 (Les Laboratoires Servier) states that the disclosed co-crystals make it possible to modify the dissolution rate of the active ingredient (i.e. agomelatine) and that the co-crystals according to their invention have a dissolution rate that is accelerated or delayed compared to the form available on the market which is described in patent specification EP 1 564 202 and marketed under the trade mark Valdoxan®.

However, this was not observed when developing pharmaceutical formulation comprising co-crystal of agomelatine and suitable organic acid such as citric acid. A pharmaceutical formulation in the form of tablet was developed with the tablet shape, the tablet size, excipient types, impurity profile and physic-chemical stability comparable to that of Valdoxan®. No modified dissolution rate was observed. On contrary, the dissolution profile of such tablet comprising agomelatine co-crystal was similar to the marketed formulation Valdoxan®.

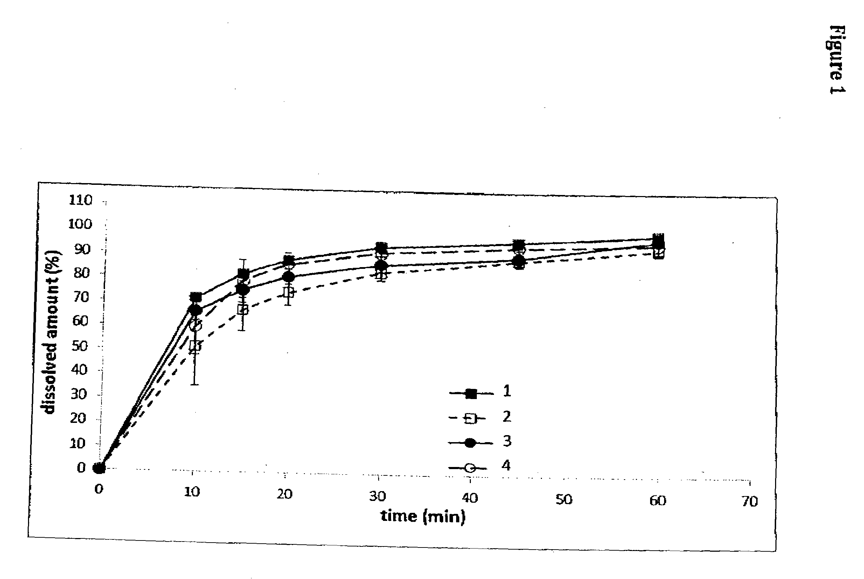

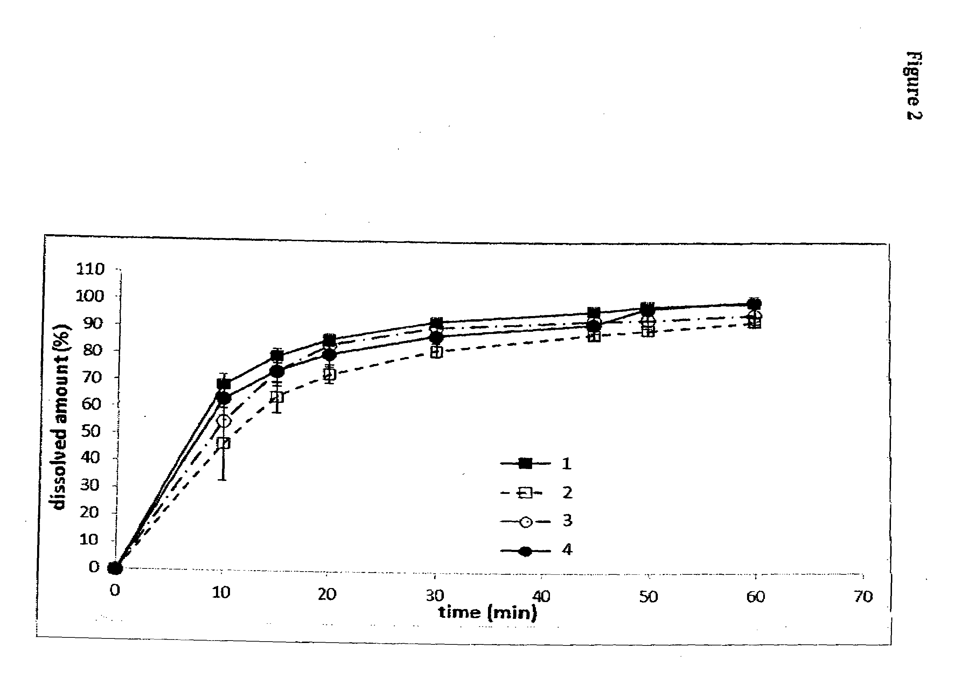

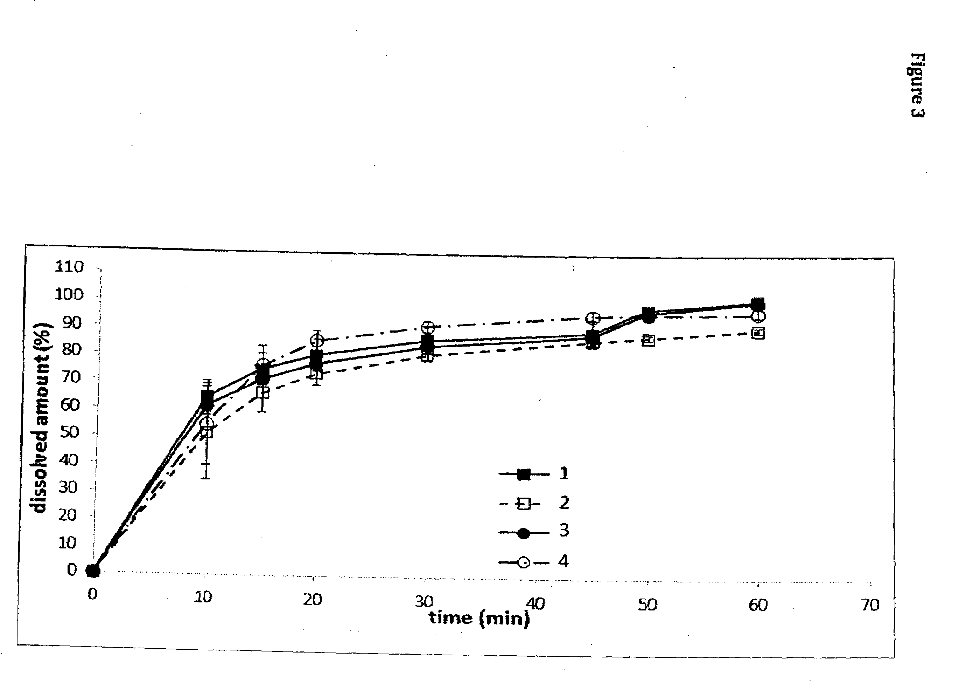

In Figures 1-3 are shown dissolution profiles at pH 2.45 [Fig. 1], at pH 4.5 (Fig. 2) and at pH 6.8 (Fig. 3), in each case for two batches of marketed product Valdoxan® and for two batches of formulation according to this invention. The dissolution method is described as follows: 900 ml of dissolution medium, paddles, 50 rpm for 45 minutes then 150 rpm. Three different media tested: pH 2 [0.01 M HCl], pH 4.5 (phosphate buffer) and pH 6.8 (phosphate buffer).

As stated above, the results of the dissolution test showed no significant differences between tested media for both products.

The dissolution of active pharmaceutical ingredients is an important *in-vitro* characteristic. In order to cross biological membranes or in order to be absorbed, the active ingredient has to be dispersed in the molecular state in aqueous media (that is to say, dissolved) at the absorption site. In the pharmaceutical industry, active pharmaceutical ingredients dissolution testing is routinely used to provide critical *in-vitro* active pharmaceutical ingredients release information for drug development, i.e. to predict *in-vivo* drug release profiles.

### In -vivo pharmacokinetic study

The therapeutic effect of active substances is generally dependent on their interaction with receptors. Strength of the effect is usually dependent on the concentration of the substance on receptor. The substance is often delivered to the receptor by blood circulation as other substances necessary for metabolism of the cell. Thus, in simplified model, the concentration of the active substance in blood plasma determines the therapeutic effect of the drug. By measuring the concentration in plasma, the strength of the therapeutic effect can be estimated.

In typical pharmacokinetic study the substance is administered and the concentration in plasma is measured in several time points. Then pharmacokinetic curve is plotted showing the concentration in plasma in time. The most commonly used parameters to measure the abundance of the substance in plasma are Cₘₐₓ and AUC₀₋ₜ.

Cₘₐₓ is the highest concentration reached. It reflects both how fast and how much the given substance is absorbed.

AUC₀₋ₜ is the area under the pharmacokinetic curve from time of administration till the last time-point measured. It reflects how much substance is absorbed during that time.

It was very surprising that despite having similar *in-vitro* dissolution profiles, the developed drug product comprising agomelatine co-crystal with citric acid had significantly different *in-vivo* pharmacokinetics compared to the original product Valdoxan®, as it was shown by the results of the pharmacokinetic study.

The *in-vivo* pharmacokinetic experiment performed to support this application clearly demonstrated that the bioavailability of prototype with agomelatine co-crystal (Batch No. 541012) was by 35 - 40% higher compared to Valdoxan® with agomelatine form-II (Batch. No. 911758). Similar increase was observed both for Cₘₐₓ and AUC₀₋ₜ. As described in previous sections the formulation of tested products was similar. The study was performed on 24 healthy subjects, in standardized fasted conditions, in fully-replicated cross-over design.

Thus, enhanced co-crystal form of agomelatine would allow the agomelatine dose reduction without negative impact on therapeutic effect. The reduced dose of agomelatine will be within 15 to 22 mg, preferably within 16 to 20 mg and more preferably it will be 18 mg once daily taken orally. Similarly to the marketed product Valdoxan®, if there is no improvement of symptoms, the dose may be doubled.

Lower dose will interfere less with metabolism of CYP 1A2 substrates both endogenous signalling molecules and exogenous substances (e.g. other therapeutic substances). The risk of disruption of endogenous signalling or concomitant pharmacotherapy is reduced by the present invention.

### Pharmaceutical formulations

Similarly to the marketed product Valdoxan®, the formulation according to the invention comprising agomelatine co-crystal with citric acid in the molar ratio 1:1 (i.e. "tested formulation") was in the form of a film-coated tablet in the presented invention. The tablet shape, the tablet size, excipient types, impurity profile and physico-chemical

stability of these products were identical. As shown in the table 1, excipient types and their amounts are similar for both, the tested formulation and Valdoxan® formulation. The filler content is decreased by the concentration of co-crystal former citric acid in the tested formulation and the disitegrant content is comparable. The slight differences in the binder, glidant and lubricant contents came out from the technological physico-chemical properties of the agomelatine co-crystal and as shown by the dissolution profiles (Figures 1-3) they had no impact on the dissolution rate of the tested formulation in comparison to the Valdoxan® formulation. Based on the similarity of the dissolution profiles of the tested formulation with the Valdoxan® formulation it can be implied that the high bioavailability observed in the *in-vivo* testing is brought by the difference in the physic-chemical state of the active ingredients (agomelatine co-crystal versus agomelatine polymorphic form II).

**Table 1: Qualitative and quantitative composition of the tested formulation comprising agomelatine co-crystal with citric acid as co-crystal former and of the formulation of Valdoxan® comprising the polymorphic form II of agomelatin**

| | % weight in tablet core | |
|---|---|---|
| Substance / excipient type | tested formulation | Valdoxan^{®}** |
| agomelatine* | 19.2 | 19.3 |
| co-crystal former* | 15.2 | - |
| filler | 45.8 | 65.8 |
| disintegrant | 6.0 | 5.2 |
| binder | 3.1 | 6.2 |
| glidant | 5.9 | 0.8 |
| lubricant | 4.8 | 2.7 |
| **Total** | **100.0** | **100.0** |

| | | |
|---|---|---|
| *present together as a parts of co-crystal in the drug product **approximate values determined by analysis of commercially available Valdoxan® tablets | | |

Thus, any formulation comprising agomelatine in the form of co-crystals with an organic acid, such as citric acid, maleic acid or benzenesulfonic acid, preferably with citric acid, still more preferably with citric acid in the molar ratio 1:1 will exhibit the enhanced bioavailability of agomelatine. Such formulation is demonstrated but not limited to the composition shown in the table 2. The formulation is preferably in the form of film-coated tablet, however, other solid oral forms, such as capsules or sachets filed with a powder or granulate, orodispersible tablets, effervestcent tablets etc., can be prepared as well.

**Table 2: General composition comprising agomelatine in the form of co-crystals**

| Substance/Excipient type | Amount range in the formulation (weight % in the composition) |
|---|---|
| agomelatine co-crystal | 10-50 |
| Fillers | 30-80 |
| Binders | 1-5 |
| Glidants | 0.5-8 |
| Disintegrants | 2-10 |
| Lubricants | 0.5-7 |
| Film-forming agents | up to 5 |
| Plasticizers | up to 2 |
| Pigments | up to 1 |

Examples of suitable excipients hereinbelow illustrate the invention but do not limit it.

**Fillers:** Microcrystalline celullose, silicified microcrystalline cellulose, lactose, lactose monohydrate, sorbitol, mannitol, maltitol, xylitol, lactitol, calcium phosphate, starch, sucrose, glucose, calcium carbonate and any combination threof.

**Binders:** Polyvinylpyrrolidones, copovidone, hypromellose, hyprollose or other cellulose esters, cellulose ethers, pregelatinized starch and any combination threof.

**Disintegrants:** Croscarmellose sodium (sodium starch glycolate), crospovidone, starch and any combination thereof.

**Glidants:** Colloidal anhydrous silica, talc, magnesium carbonate, stearic acid or its metallic salts such as magnesium stearate, sodium stearyl fumarate, magnesium palmitate, magnesium oleate, hydrogenated vegetable oil, hydrogenated castor oil, talc, macrogols (polyethylene glycoles) of different molecular weights and any combination threof.

**Lubricants:** Sodium stearyl fumarate, stearic acid, stearic acid salts such as magnesium stearate, calcium stearate, magnesium palmitat, magnesium oleate, hydrogenated vegetable oil, hydrogenated castor oil, talc, colloidal anhydrous silica, macrogols (polyethylene glycoles), and any mixtures thereof.

**Film-forming agents:** Hypromellose, hyprollose, shellac

**Plasticizers:** Macrogols, glycerol

**Pigments:** Ferric oxides (yellow, red, black), titanium dioxide, riboflavin, quinoline yellow, sunset yellow

The formulations may voluntary comprise surfactants, e.g. sodium lauryl sulfate, polyoxyethylene sorbitan fatty acid esters, polyoxyethylene alkyl ethers, sorbitan fatty acid esters, polyethylene glycols, sugar esters of fatty acids and glycerides of fatty acids or mixtures thereof.

The formations can be prepared by any method known in the art, i.e. direct compression, wet granulation, dry granulation, roller compaction etc.

## Claims

1. A pharmaceutical formulation, comprising agomelatine in the form of agomelatine co-crystal with a co-crystal former selected from the group of organic acids in an amount corresponding to 15 - 22 mg agomelatine dose per one dosage unit and at least one pharmaceutically acceptable excipient for effective treatment of diseases responsive to therapy by agomelatine.

2. A pharmaceutical formulation according to Claim 1, wherein the co-crystal former is selected from the group of citric acid, maleic acid and benzenesulfonic acid.

3. A pharmaceutical formulation according to Claim 2, wherein the co-crystal former is citric acid.

4. A pharmaceutical formulation according to Claim 3, wherein the co-crystal former is citric acid and the molar ratio of agomelatine to the co-crystal former is 1:1.

5. A pharmaceutical formulation according to any one of the preceding claims, wherein the amount of agomelatin co-crystal corresponds to 16 - 20 mg agomelatine.

6. A pharmaceutical formulation according to any one of the preceding claims, wherein the amount of agomelatin co-crystal corresponds to 18 mg agomelatine.

7. A pharmaceutical formulation according to any one of the preceding claims, wherein the disease responsive to therapy by agomelatine are major depressive episodes.

8. A pharmaceutical formulation according to claims 1-6, wherein the disease responsive to therapy by agomelatine is generalized anxiety disorder.

9. A pharmaceutical formulation according to claims 1-6, wherein the disease responsive to therapy by agomelatine are sleep disorders among depressed patients.

10. A pharmaceutical formulation according to claims 1-6, wherein the disease responsive to therapy by agomelatine is periventricular leukomalacia

11. A pharmaceutical formulation according to claims 1-6, wherein the disease responsive to therapy by agomelatine is Smith-Magenis syndrome.

12. A pharmaceutical formulation according to any one of the preceding claims, wherein it is administered in one daily dose.

13. A pharmaceutical formulation according to claims 1-11, wherein the formulation is administered in two daily doses.
